# EUROPEAN PATENT SPECIFICATION

(11) **EP 4 506 911 B1**
(45) Date of publication and mention of the grant of the patent: **15.07.2026**
(21) Application number: 23779599.2
(22) Date of filing: 15.03.2023
(51) Int. Cl.: G06V 40/50, G06T 7/00, G06V 40/16, G06F 3/16, G06F 3/01, G06F 21/32, A61B 5/1171, G06V 40/60, A61B 5/00

(54) **MOBILE BODY, CONTROL DEVICE, AND CONTROL METHOD**
MOBILER KÖRPER, STEUERUNGSVORRICHTUNG UND STEUERUNGSVERFAHREN
CORPS MOBILE, DISPOSITIF DE COMMANDE ET PROCÉDÉ DE COMMANDE

(30) Priority: 01.04.2022 JP 2022062064
(43) Date of publication of application: 12.02.2025
(73) Proprietor: Sony Group Corporation, Tokyo 108-0075 (JP)
(72) Inventor: KANEKO, Emika, Tokyo 108-0075 (JP); FUJIMOTO, Yoshihide, Tokyo 108-0075 (JP); YAMAZAKI, Seiichi, Tokyo 108-0075 (JP); HOSOI, Takafumi, Tokyo 108-0075 (JP); KARINO, Takatoshi, Tokyo 108-0075 (JP); NAGAE, Mika, Tokyo 108-0075 (JP); TSUCHIYA, Yuto, Tokyo 108-0075 (JP)
(74) Representative: MFG Patentanwälte Meyer-Wildhagen Meggle-Freund Gerhard PartG mbB
(86) International application number: PCT/JP2023/010003
(87) International publication number: WO 2023/189581

(56) References cited:
- WO-A1-2017/217314
- WO-A1-2018/180666
- JP-A- 2000 259 834
- JP-A- 2003 141 541
- JP-A- 2013 182 325
- JP-A- 2015 090 662
- AMANDA H A H ET AL: "Interactive robotic head for neck exercises", 2015 MORATUWA ENGINEERING RESEARCH CONFERENCE (MERCON), IEEE, 7 April 2015 (2015-04-07), pages 165 - 170, XP032780658, DOI: 10.1109/MERCON.2015.7112339

## Description

### Technical Field

The present disclosure relates to a moving object and a control method, and more particularly, to a moving object and a control method capable of performing intuitive face registration.

### Background Art

In recent years, in various electronic devices such as a smartphone, a face authentication method has been widely used in which face authentication is performed based on face information of a user registered in advance, and a lock is released when the face is identified as a face of the user itself.

For example, Patent Literature 1 discloses a face recognition apparatus that identifies an input face image by evaluating similarity with a registered face image registered in a registered face group selected based on an input result of an image or a sound, and confirms that the identified input face image is a person itself of the registered face image.
AMANDA H A H ET AL: "Interactive robotic head for neck exercises", 2015 MORATUWA ENGINEERING RESEARCH CONFERENCE (MERCON), IEEE, DOI: 10.1109/MERCON.2015.7112339 discloses a humanoid robotic head which acts as an exercise instructor.
JP 2013 182325 A discloses a face registration device, program and face registration method.

### Citation List

### Patent Literature

Patent Literature 1: Japanese Patent Application Laid-open No. 2004-302644

### Disclosure of Invention

### Technical Problem

In the related art, in face registration processing in which face information of a user is registered in advance, a direction of a face of the user is guided by using characters and images displayed on a display, but it is required to perform more intuitive face registration.

The present disclosure is made in view of such circumstances, so that intuitive face registration can be performed.

### Solution to Problem

According to a first aspect, the present invention provides a moving object in accordance with independent claim 1. According to a second aspect, the present invention provides a control method in accordance with independent claim 6. Further aspects are set forth in the dependent claims, the drawings and the following description.

A moving object according to an aspect of the present disclosure include a gesture control section that controls a gesture driving that expresses a movement of a face of a user by a gesture at the time of a streaming capture in subsequent processing of a tutorial of face registration processing that
registers the face of the user in advance; and a guidance sound control section that controls an output of a gesture guidance sound in accordance with a gesture along with the gesture driving.

A control method according to an aspect of the present disclosure includes controlling a gesture driving that expresses a movement of a face of a user by a gesture at the time of a streaming capture in subsequent processing of a tutorial of face registration processing that registers the face of the user in advance; and controlling an output of a gesture guidance sound in accordance with a gesture along with the gesture driving.

In an aspect of the present disclosure, a gesture driving that expresses a movement of a face of a user by a gesture is controlled at the time of a streaming capture in subsequent processing of a tutorial of face registration processing that registers the face of the user in advance; and an output of a gesture guidance sound in accordance with a gesture along with the gesture driving is controlled.

### Brief Description of Drawings

[Fig. 1] A diagram showing an example of a usage state of a moving object to which the present technology is applied.
[Fig. 2] A diagram for describing an example of a gesture guidance sound and a gesture driving.
[Fig. 3] A block diagram showing a configuration example of an embodiment of a moving object.
[Fig. 4] A diagram for describing feature vectors and a center vector.
[Fig. 5] A diagram for describing a threshold value.
[Fig. 6] A flowchart for describing face registration processing.
[Fig. 7] A block diagram showing an example of a configuration of an embodiment of a computer to which the present technology is applied.

### Mode(s) for Carrying Out the Invention

Hereinafter, specific embodiments to which the present technology is applied will be described in detail with reference to the drawings.

### <Usage Example of Moving Object>

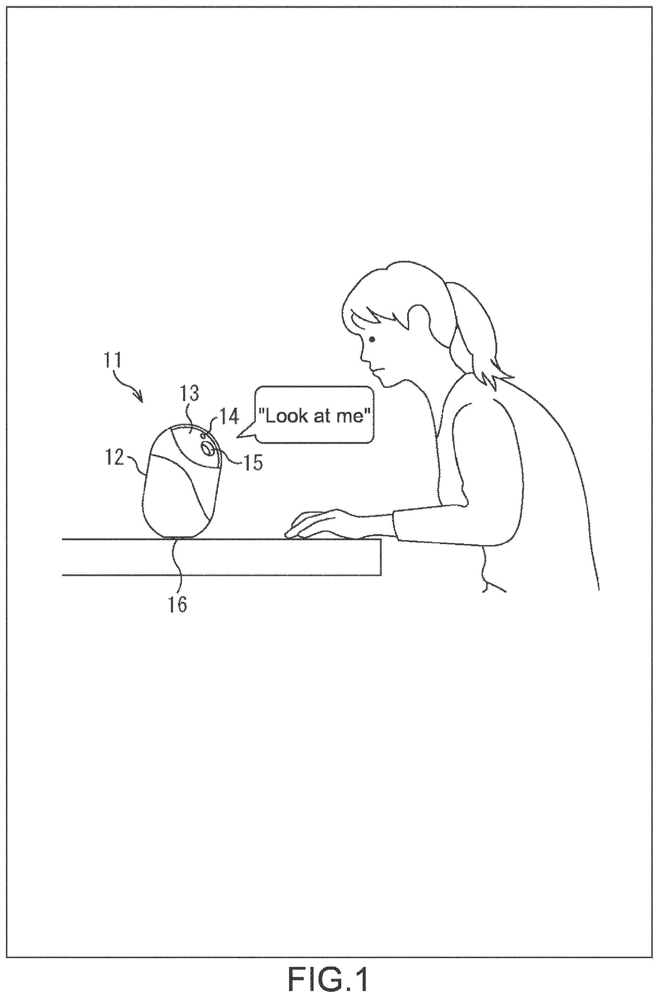

Fig. 1 is a diagram for describing a usage state of a moving object to which the present technology is applied. Fig. 1 shows an example of a state in which a user uses a moving object 11 placed on a table as viewed from a lateral direction.

For example, the moving object 11 is an agent-type robot apparatus capable of performing autonomous traveling, and enables communication with the user to be realized more naturally and effectively. In addition, the moving object 11 is, for example, a small robot configured with a size and a weight such that the user can easily lift with one hand.

The moving object 11 has a configuration in which a hemispherical face portion 13 is provided on an upper portion of a main body 12 of a long ellipsoid in a longitudinal direction, a camera 14 and an eye portion 15 are provided on a front side (a side facing the right direction in Fig. 1) of the face portion 13, and a tire 16 is provided on a bottom surface of the main body 12.

The face portion 13 is configured to be able to freely change a direction in the up-down direction and the left-right direction by a drive mechanism built in the moving object 11.

The camera 14 captures an image in the direction toward the front of the face portion 13, and acquires a still image or a moving image. For example, as shown in Fig. 1, when the user faces the face portion 13, the camera 14 can acquire a face image by capturing a face of the user.

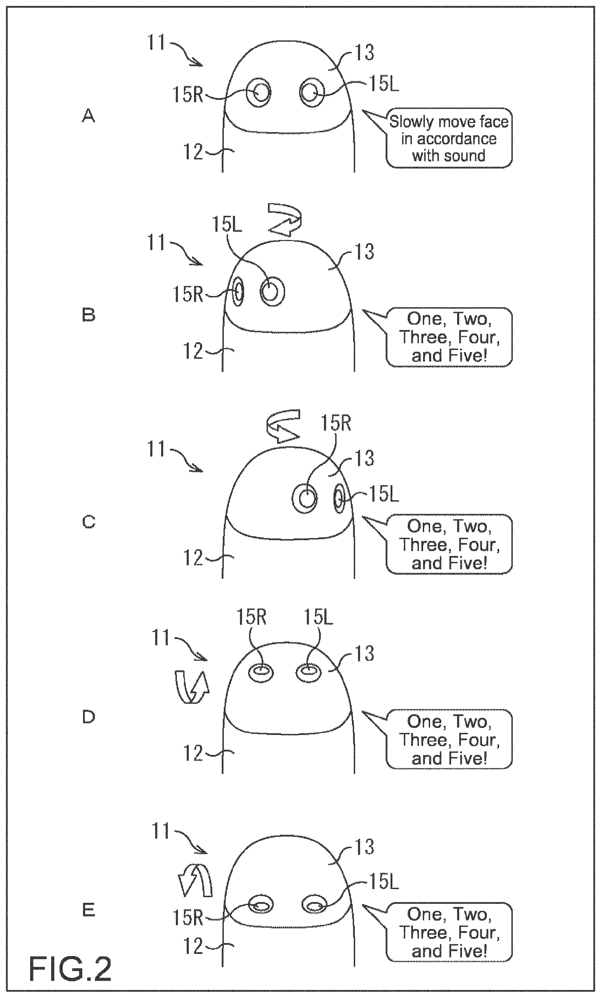

The eye portion 15 includes, for example, an LED (Light Emitting Diode), an organic EL (Electro Luminescence) or the like, and can express a line of sight, a blink, or the like. In Fig. 1, only one eye portion 15 is shown, but as shown in Fig. 2, two eye portions 15L and 15R are provided side by side with the face portion 13 viewed from the front.

The tire 16 can freely rotate by the drive mechanism built in the moving object 11, and realizes moving operations such as an advance movement, a backward movement, a turning, and rotation of the moving object 11.

The moving object 11 configured as described above can register face information of the user in a face database in advance, and can realize communication suitable for each user by performing face authentication processing when the user uses.

In a case where the face authentication processing is performed, the moving object 11 outputs, for example, a guidance sound of "look at me" from a speaker (not shown). In response to this, when the user brings the face closer to the face portion 13 of the moving object 11, the moving object 11 acquires the face information from the face image obtained by capturing the face of the user by the camera 14. Then, the moving object 11 can identify the face of each user by performing the face authentication processing that evaluates similarity between the face information of the user and each of a plurality of face information registered in the face database.

For example, in the face authentication processing using feature vectors as the face information, a distance (cos distance (similarity), Euclidean distance, or the like) between each identification target feature vector and each registered feature vector is calculated on a one-to-one basis using the feature vectors acquired from the face of the user captured by the camera 14 as an identification target. Then, in the face authentication processing, it is possible to identify that the face of the registered feature vector of which distance is equal to or larger than a predetermined threshold value and the face of the user captured by the camera 14 are of the same person.

Incidentally, in face registration processing that registers the face information of the user in advance, it is necessary to guide the user so as to change the direction of the face in the front, the right direction, the left direction, the upward direction, and the downward direction toward the camera. For example, when the face registration processing is performed in a smartphone, the user is guided to change the direction of the face by using characters and images displayed on a display.

On the other hand, the moving object 11 is configured to perform intuitive face registration processing without using the display, that is, the face registration processing in which the user can easily understand that the user changes the direction of the face by a tutorial using a gesture guidance sound and a gesture driving.

With reference to Fig. 2, an example of the gesture guidance sound and the gesture driving in the tutorial when the moving object 11 performs the face registration processing will be described.

For example, the gesture driving expresses a movement (speed and direction) of the face of the user at the time of a streaming capture by a gesture of the face portion 13 of the moving object 11, and the gesture guidance sound outputs a constant rhythm corresponding to the speed of the face portion 13 of the moving object 11 in accordance with the gesture.

First, as shown in A of Fig. 2, the moving object 11 outputs a preliminary guidance sound "Slowly move the face in accordance with the sound" with the face portion 13 facing the front, explains how to move the face, and then starts the tutorial.

For example, in the tutorial, as shown in B of Fig. 2, the moving object 11 outputs the gesture guidance sound with the constant rhythm of " One, Two, Three, Four, and Five!" and also performs the gesture driving to rotate the face portion 13 to face the right side at a constant speed. Similarly, as shown in C of Fig. 2, the moving object 11 outputs the gesture guidance sound with the constant rhythm of " One, Two, Three, Four, and Five!" and also performs the gesture driving to rotate the face portion 13 so as to face the left side at the constant speed.

In addition, as shown in D of Fig. 2, the moving object 11 outputs the gesture guidance sound with the constant rhythm of " One, Two, Three, Four, and Five!" and also performs the gesture driving to rotate the face portion 13 so as to face upward at the constant speed. Similarly, as shown in E of Fig. 2, the moving object 11 outputs the gesture guidance sound with the constant rhythm of " One, Two, Three, Four, and Five!" and also performs the gesture driving to rotate the face portion 13 so as to face downward at the constant speed.

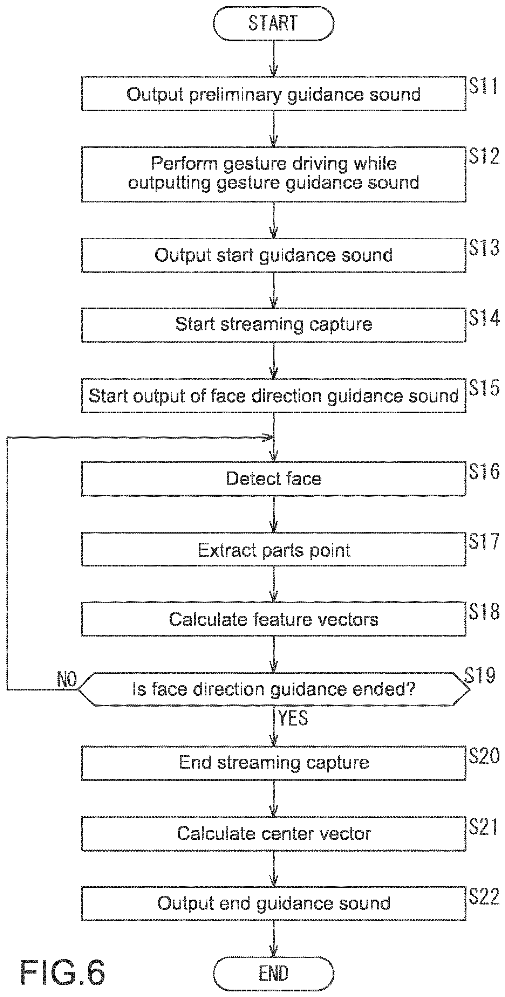

After the tutorial is ended, the moving object 11, as described later with reference to the flow chart of Fig. 6, starts the streaming capture, similar to the gesture guidance sound at the time of the gesture driving, outputs a face direction guidance sound with the constant rhythm of " One, Two, Three, Four, and Five!" that guides the movement of the face of the user. At this time, the moving object 11 performs the streaming capture of the face of the user while the face portion 13 is fixed in front, and guides the user to change the direction of the face in order in the right direction, the left direction, the upward direction, and the downward direction. In addition, the moving object 11 guides the user so that the face of the user always faces the front while changing the direction of the face in the right direction, the left direction, the upward direction, and the downward direction, respectively.

Note that in the tutorial, it is not necessary to perform all of the gestures in the four directions of the right direction, the left direction, the upward direction, and the downward direction, and it is only necessary to perform the gesture in at least one direction. For example, the moving object 11 may perform the gesture of one direction in the left-right direction and one direction in the up-down direction in the tutorial.

### <Configuration Example of Moving Object>

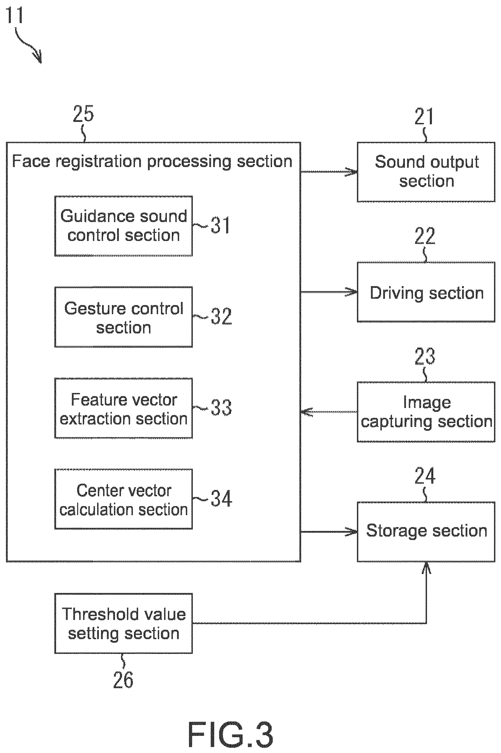

Fig. 3 is a block diagram showing a configuration example of an embodiment of the moving object.

As shown in Fig. 3, the moving object 11 includes a sound output section 21, a driving section 22, an image capturing section 23, a storage section 24, a face registration processing section 25, and a threshold value setting section 26. The face registration processing section 25 performs the face registration processing and includes a guidance sound control section 31, a gesture control section 32, a feature vector extraction section 33, and a center vector calculation section 34.

The sound output section 21 includes, for example, a speaker or the like, and outputs the guidance sound required for guidance when the face registration processing is performed in accordance with control by the guidance sound control section 31.

The driving section 22 includes, for example, a motor or the like, and performs the gesture driving in which the face section 13 is rotated as described with reference to Fig. 2 in accordance with control by the gesture control section 32 to move the face section 13 so as to face in the right direction, the left direction, the upward direction, and the downward direction, respectively.

The image capturing section 23 includes, for example, an imaging element included in the camera 14, can acquire an image by capturing an image of a subject in front of the face section 13, acquires the face image by, for example, streaming capturing the face of the user, and supplies the face image to the feature vector extraction section 33.

The storage section 24 includes, for example, a nonvolatile memory such as a flash memory, and registers a center vector calculated by the center vector calculation section 34 in the face registration processing in the face database.

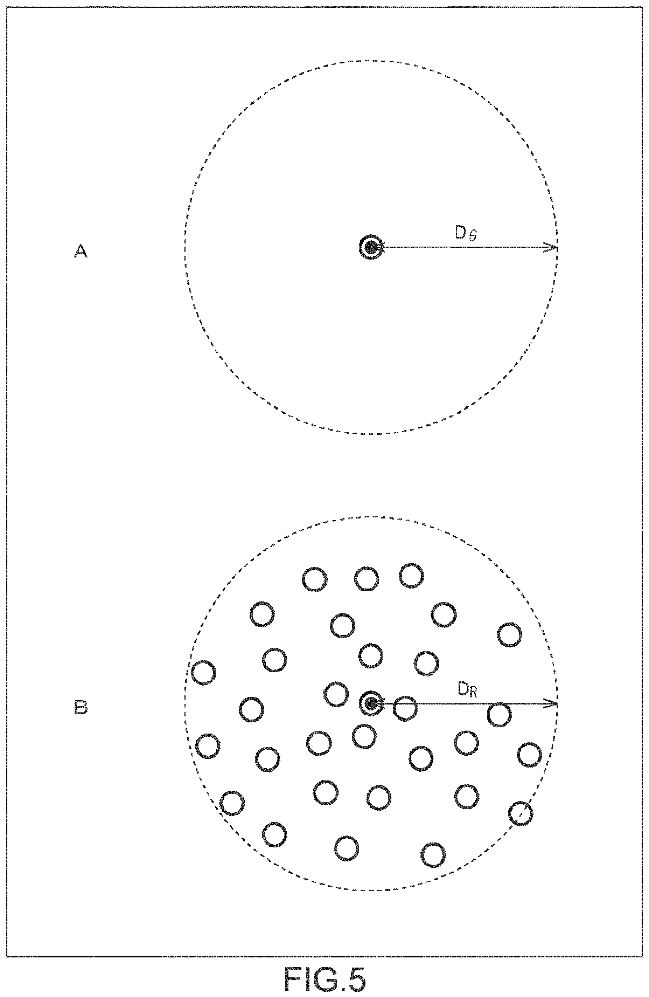

The threshold value setting section 26 sets a threshold value used in the face authentication processing that evaluates similarity with the center vector registered in the face database, and stores the threshold value in the storage section 24. The threshold value set by the threshold value setting section 26 will be described later with reference to Fig. 5.

During the tutorial, the guidance sound control section 31 controls an output of the gesture guidance sound in accordance with the gesture of the face section 13 of the moving object 11, that is, the output of the preliminary guidance sound and the gesture guidance sound as described with reference to Fig. 2. Furthermore, the guidance sound control section 31 controls the output of a start guidance sound, the face direction guidance sound, an end guidance sound, or the like as described with reference to a flowchart of Fig. 6 to be described later, and causes the sound output section 21 to output the guidance sound.

During the tutorial, the gesture control section 32 controls the movement of the face of the user at the time of the streaming capture, i.e., the gesture driving that expresses the speed and the direction at which the user moves the face by the gesture of the face section 13 of the moving object 11. That is, as described with reference to Fig. 2, the gesture control section 32 controls the driving section 22 so as to perform the gesture driving for rotating the face section 13 so as to face the right direction, the left direction, the upward direction, and the downward direction at the constant speed in accordance with the rhythm of the gesture guidance sound.

The feature vector extraction section 33 extracts a plurality of feature vectors from the face images of various angles acquired by the streaming capture by the image capturing section 23, and supplies the feature vectors to the center vector calculation section 34.

The center vector calculation section 34 calculates the center vector that is the center of all the feature vectors supplied from the center vector calculation section 34.

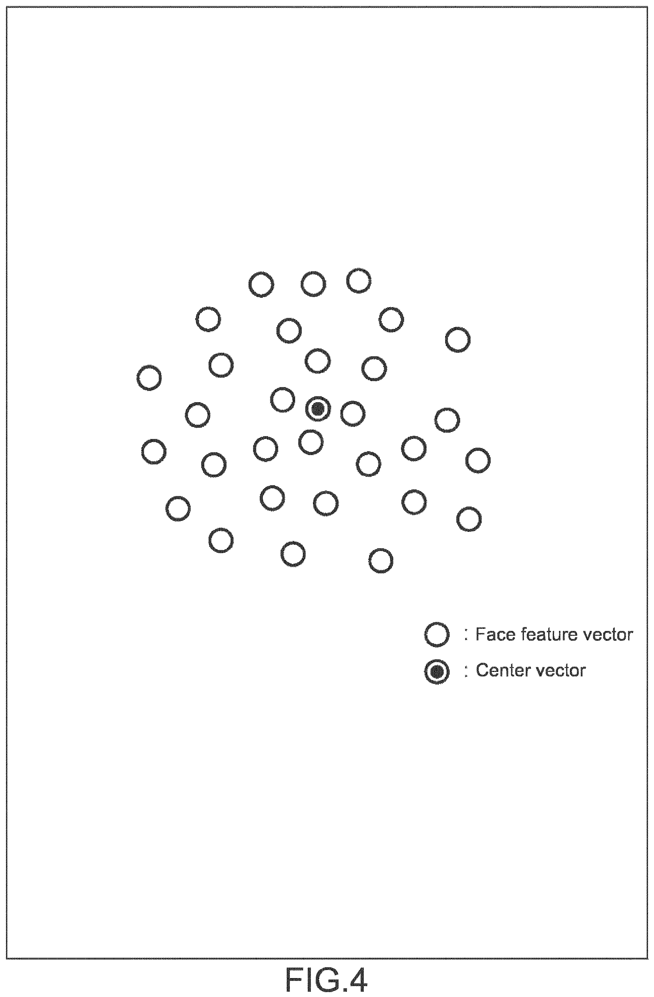

Here, the feature vectors and the center vector will be described with reference to Fig. 4.

As described above, the moving object 11 performs the streaming capture in the face registration processing, and the feature vector extraction section 33 extracts the plurality of feature vectors from the face images of various angles acquired by the streaming capture. Although Fig. 4 shows an image of the plurality of feature vectors, in fact, the feature vectors are vectors on a 512-dimensional hypersphere.

Then, the center vector calculation section 34 calculates the center vector using all the face images acquired by the streaming capture. That is, the result is stabilized by using the center of the feature vectors extracted from the face images of various angles. Note that the center vector calculation section 34 may calculate the center vector at a time point when a predetermined number (for example, 50) of feature vectors are accumulated. Furthermore, the feature vector extraction section 33 is learned in advance so that the features of the face of the same person are close and the features of the faces of different persons are far away.

Furthermore, as shown in Fig. 5, the threshold value setting section 26 sets any one of an aimed threshold value D_{θ}, a maximum distance threshold value D_{R}, and a maximum value of the aimed threshold value D_{θ} and the maximum distance threshold value D_{R} as the threshold value to be used in the face-authentication processing. For example, the aimed threshold value D_{θ} is a value determined at the time of designing the moving object 11, and the maximum distance threshold value D_{R} is a value corresponding to a distance to the feature vector at the farthest position centered on the center vector.

By using the threshold value set in this way, the moving object 11 can verify and implement the face authentication processing relatively easily, and can reduce a processing load.

When the face authentication processing is performed, the moving object 11 calculates a distance between the feature vector extracted by the feature vector extraction section 33 and the registered center vector, and sets the center vector with the distance to be the closest and falling within the range of the threshold value set by the threshold value setting section 26 to be the same.

Here, the moving object 11 is equipped with a feature extractor learned so as to minimize the distance between the center vector of each face class or a representative vector and the feature vector of the same face class. That is, the feature extractor distributes the feature vectors of faces of various angles of a certain person so as to spread around the center vector or a representative vector. Therefore, the center of the feature vector of the face images of various angles acquired by the streaming capture can capture an approximate center of distribution of the feature vectors of the face. In addition, since the feature vectors output from the feature extractor are normalized and a constraint that the feature vectors exist on the hypersphere is added, it is possible to avoid being affected by lengths of the vectors, and thus it is expected that the center of the feature vectors of the face images collected by the streaming capture is stabilized. On the other hand, in the feature extractor learned so as not to minimize the distance between the center vector of each face class or the representative vector and the feature vectors of the same face class, but to simply make closer the distance between the feature vectors of the same face and to make away the distance between the feature vectors of different faces, there is no guarantee that a shape of the distribution spreads concentrically (hypersphere rather than circle because it is multidimensional), and it is not possible to expect the accuracy in which face identification may be performed using the center of the feature vectors of the face images collected by the streaming capture.

### <Processing Example of Face Registration Processing>

The face registration processing performed by the face registration processing section 25 will be described with reference to the flowchart shown in Fig. 6.

For example, when the user uses the moving object 11 for the first time and talks "let's be friends", the face registration processing is started as a result of the speech recognition processing for the talk.

In Step S11, the guidance sound control section 31 controls the output of the preliminary guidance sound and causes the sound output section 21 to output the preliminary guidance sound. For example, the sound output section 21 outputs the preliminary guidance sound "Let just take a moment to recognize your face to commemorate our encounter" and outputs the preliminary guidance sound "I learn at a variety of angles." Thus, an explanation will be given to the user to capture the face at the plurality of angles. When the user is the owner of the moving object 11, the name of the user is registered in advance, and the name of the user may be confirmed at the start of the face registration processing. Then, the sound output section 21 outputs the preliminary guidance sound "I'll show an example first!" to explain to the user that the tutorial is started, and the processing proceeds to Step S12.

In Step S12, the guidance sound control section 31 controls the output of the gesture guidance sound, and the gesture control section 32 controls the gesture driving. Thus, as described above with reference to Fig. 2, the tutorial is performed by the driving section 22 performing the gesture driving while the sound output section 21 outputs the gesture guidance sound.

In Step S13, the guidance sound control section 31 controls the output of the start guidance sound and causes the sound output section 21 to output the start guidance sound. For example, the sound output section 21 outputs the start guidance sound "Do you understand? Move your face as slowly as possible like this" to explain how to move the face, and outputs the start guidance sound "All light, I'm going to start memorizing your face now" to declare that capturing of the face is to be started. Then, the sound output section 21 outputs the start guidance sound of "Stare at my face" so that the user faces the front, and then the processing proceeds to Step S14.

In Step S14, the streaming capture by the image capturing section 23 is started, and the face images are sequentially supplied from the image capturing section 23 to the feature vector extraction section 33.

In Step S15, the guidance sound control section 31 controls the output of the face direction guidance sound, and starts the output of the face direction guidance sound from the sound output section 21. Thus, the sound output section 21 starts to the output of the face direction guidance sound, for example, such as "From there, turn to the right. One, Two, Three, Four, and Five!", "Look at my face again. Now, turn to the left. One, Two, Three, Four, and Five!", "Look at my face again. Now, look up. One, Two, Three, Four, and Five!", and "Look at my face again. Now, look down. One, Two, Three, Four, and Five!."

In Step S16, the feature vector extraction section 33 detects the face of the user from the face images supplied from the image capturing section 23. Here, in the feature vector extraction section 33, when the size of the detected face of the user is small, it makes difficult to detect a parts point in Step S17, and thus it does not proceed to Step S17 until the face of the user having a predetermined size or larger is detected. For example, in this case, the moving object 11 is moved by driving the tire 16 so that the face image of an appropriate size can be captured.

In Step S17, the feature vector extraction section 33 detects, from the face of the user detected in Step S16, the part points that are features of the respective parts such as eyes, nose, and mouth, and estimates the direction (yaw, pitch, roll) of the face based on the parts point. Here, when an estimated face direction is out of a specified range or when the face direction cannot be estimated, the feature vector extraction section 33 does not proceed to Step S18.

In Step S18, the feature vector extraction section 33 adjusts the position using the parts point estimated in Step S17, extracts the feature vectors of the face of the user, and supplies the feature vectors to the center vector calculation section 34.

In Step S19, the face registration processing section 25 determines whether or not a face direction guidance is ended. For example, the face registration processing section 25 determines that the face direction guidance is ended when the output of the face direction guidance sound started in Step S15 is ended, that is, when all of the guidance for directing the face of the user in the right direction, the left direction, the upward direction, and the downward direction is performed.

In Step S19, when the face registration processing section 25 determines that the face direction guidance is not ended, the processing returns to Step S16, and the same processing is repeatedly performed thereafter. On the other hand, in Step S19, when the face registering processing section 25 determines that the face direction guidance is ended, the processing proceeds to Step S20.

In Step S20, the streaming capture by the image capturing section 23 is ended, and a supply of the face images from the image capturing section 23 to the feature vector extraction section 33 is stopped. At this time, the center vector calculation section 34 accumulates the plurality of feature vectors supplied from the feature vector extraction section 33 in a period in which the streaming capture is performed.

In Step S21, the center vector calculation section 34 obtains the center vector by calculating the center of the plurality of feature vectors supplied from the feature vector extraction section 33, and registers the center vector in the face database of the storage section 24.

In Step S22, the guidance sound control section 31 controls the output of the end guidance sound, and causes the sound output section 21 to output the end guidance sound. Here, the processing of Step S22 can be performed at a time required to perform the processing of Steps S20 and S21. For example, the sound output section 21 outputs, at the time required to perform the processing of Steps S20 and S21, the end guidance sound such as " All light, so I don't forget your face, so wait a little" or the end guidance sound such as "I'm remembering now, so wait a little." When the user uses the moving object 11 for the first time, the name of the user may be registered. Then, when the processing of Steps S20 and S21 is ended, the sound output section 21 outputs the end guidance sound "I remember!", and the processing is ended.

As described above, the moving object 11 can end the registration of the face information of the user by the guidance sound and the gesture driving without using the display. That is, in the tutorial, the moving object 11 performs the gesture driving in which the movement of the face of the user at the time of the streaming capture is expressed by the gesture based on the speed and the direction of the face portion 13 of the moving object 11 (movement range of face), and outputs the gesture guidance sound at the constant rhythm corresponding to the speed of the face portion 13 of the moving object 11 in accordance with the gesture, so that the user can easily grasp the way of moving the face at the time of the streaming capture. Therefore, the user can move the direction of the face without straying according to the face direction guidance sound.

In addition, in the face registration processing by the streaming capture, the moving object 11 can avoid that, for example, the feature vectors when facing other than the front face are treated as of different person because the feature vectors vary from the feature vectors of the front face. In addition, the moving object 11, due to the fact that it is not detected that it is faced in a particular direction by estimation accuracy of the face angle, can avoid that, for example, the face registration processing is not ended and repeating capturing many times until the face registration processing is ended. Then, the moving object 11 can provide a more robust and highly accurate face identification function by calculating the center vector that is the center of the plurality of feature vectors extracted from the face images of various angles and registering it in the face database. That is, the moving object 11 can improve face identification accuracy by using face images of various angles acquired by the streaming capture.

### <Example of Configuration of Computer>

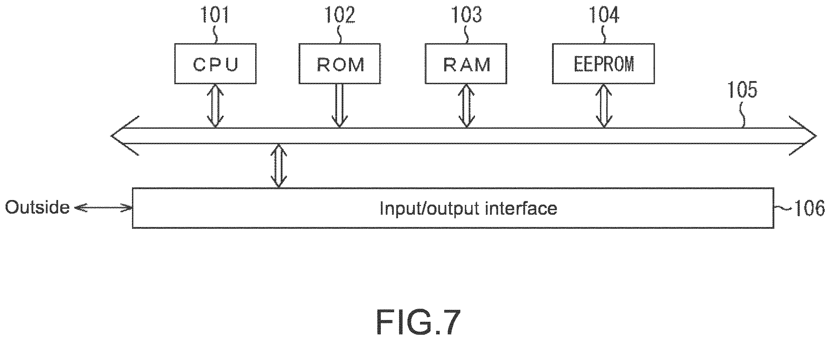

Fig. 7 is a block diagram showing an example of a configuration of hardware of a computer that executes a series of processing described above using a program.

In the computer, a CPU (Central Processing Unit) 101, a ROM (Read Only Memory) 102, a RAM (Random Access Memory) 103 and an EEPROM (Electronically Erasable and Programmable Read Only Memory) 104 are interconnected by a bus 105. Furthermore, an input/output interface 106 is connected to the bus 105, and the input/output interface 106 is connected to the outside. Note that processing of extracting feature values can be performed by a GPU (Graphics Processing Unit), a DSP (digital signal processor), an FPGA (Field Programmable Gate Array), or the like as well as the CPU.

In the computer configured as described above, the CPU 101 loads the program stored in, for example, the ROM 102 and the EEPROM 104 into the RAM 103 via the bus 105 and executes the program, thereby performing the above-described series of processing. The program executed by the computer (CPU 101) may be written in the ROM 102 in advance, or may be installed or updated in the EEPROM 104 from the outside via the input/output interface 106.

Here, the processing performed by the computer in accordance with the program in the present specification unnecessarily has to be performed in time series in order described in the flowchart. In other words, the processing performed by the computer in accordance with the program includes processing executed in parallel or individually (for example, parallel processing or object processing).

Furthermore, the program may be processed by a single computer (processor) or may be processed distributedly by a plurality of computers. Furthermore, the program may be transferred to a remote computer to be executed by the remote computer.

Furthermore, in the present specification, the system refers to a collection of a plurality of components (such as apparatuses and modules (parts)) and it does not matter whether or not all of the components are in the same housing. Thus, a plurality of apparatuses accommodated in separate housings and connected to one another via a network, and one apparatus in which a plurality of modules is accommodated in one housing are both systems.

Furthermore, for example, a configuration described as one apparatus (or processing section) may be divided and configured as the plurality of apparatuses (or processing sections). Conversely, the configuration described above as a plurality of apparatuses (or processing sections) may be collectively configured as one apparatus (or processing section). In addition, a configuration other than the above-described configurations may be added to the configuration of each apparatus (or each processing section). Furthermore, if the configuration and operation of the entire system are substantially the same, a part of the configuration of a certain apparatus (or processing section) may be included in the configuration of another apparatus (or other processing section).

For example, the present technology may also have a configuration of cloud computing in which one function is shared to be cooperatively processed by the plurality of apparatuses via a network.

Furthermore, for example, the above-described program can be executed in any apparatus. In this case, the apparatus may have a necessary function (such as a function block) so that necessary information can be obtained.

Furthermore, the respective steps described using the flowchart described above may be executed by one apparatus or may be shared and executed by the plurality of apparatuses. Moreover, when one step includes the plurality of processing, the plurality of processing included in the one step may be executed by one apparatus or may be shared and executed by the plurality of apparatuses. In other words, a plurality of processing included in one step may be executed as a plurality of steps. Conversely, the processing described as the plurality of steps may be collectively executed as one step.

Note that, as to the program executed by the computer, steps describing the program may be executed in time series in order described in the present specification, or may be executed in parallel or individually at necessary timing such as when a call is made. That is, as long as there is no inconsistency, the processing of respective steps may be executed in an order different from the above-described order. Furthermore, the processing of the steps of describing the program may be executed in parallel with the processing of the other program, or may be executed in combination with the processing of the other program.

Note that the present technology described in the present specification can be implemented independently as long as there is no inconsistency. It should be appreciated that any plurality of the present technologies may be used in combination. For example, some or all of the present technology described in any of the embodiments may be implemented in combination with some or all of the present technology described in the other embodiment. Also, some or all of any of the above-described present technology may be implemented in combination with other technology not described above.

## Claims

1. A moving object (11) capable of performing intuitive face registration, comprising:
a gesture control section (32) configured to control a gesture driving that expresses a movement of a face of a user by a gesture at the time of a streaming capture in subsequent processing of a tutorial of face registration processing that registers the face of the user in advance;
a guidance sound control section (31) configured to control an output of a gesture guidance sound in accordance with a gesture along with the gesture driving,
wherein the guidance sound control section (31) is configured to control an output of a face direction guidance sound that guides the movement of the face of the user in the streaming capture performed after the tutorial;
a feature vector extraction section (33) configured to extract a plurality of feature vectors from a face image of the user of various angles acquired by changing the direction at which the user is guided to move the face in accordance with guidance of the face direction guidance sound when the streaming capture is performed; and
a center vector calculation section (34) configured to calculate a center vector that is a center of the plurality of feature vectors and register the center vector in a face database.

2. The moving object (11) according to claim 1, wherein the gesture control section (32) is configured to express a speed and a direction at which the user is guided to move the face at the time of the streaming capture by a gesture of a face portion (13) of the moving object (11).

3. The moving object (11) according to claim 2, wherein the guidance sound control section (31) is configured to output a constant rhythm corresponding to the speed as the gesture guidance sound.

4. The moving object (11) according to claim 1, further comprising:
a threshold value setting section (26) configured to set a threshold value used in face authentication processing that evaluates similarity with the center vector registered in the face database.

5. The moving object (11) according to claim 4, wherein the threshold value setting section (26) is configured to set, as the threshold value, any one of a first threshold value which is a value determined at the time of designing, a second threshold value corresponding to a distance to the feature vector at a farthest position centered on the center vector, and a third threshold value which is a maximum value of the first threshold value and the second threshold value.

6. A control method capable of performing intuitive face registration, comprising:
controlling a gesture driving that expresses a movement of a face of a user by a gesture at the time of a streaming capture in subsequent processing of a tutorial of face registration processing that registers the face of the user in advance;
controlling an output of a gesture guidance sound in accordance with a gesture along with the gesture driving,
wherein the guidance sound control section (31) is configured to control an output of a face direction guidance sound that guides the movement of the face of the user in the streaming capture performed after the tutorial;
extracting a plurality of feature vectors from a face image of the user of various angles acquired by changing the direction at which the user is guided to move the face in accordance with guidance of the face direction guidance sound when the streaming capture is performed; and
calculating a center vector that is a center of the plurality of feature vectors and register the center vector in a face database.

## Patentansprüche

1. Sich bewegendes Objekt (11), das in der Lage ist, eine intuitive Gesichtsregistrierung durchzuführen, umfassend:
einen Gestensteuerbereich (32), der konfiguriert ist, um ein Gestentreiben zu steuern, das eine Bewegung eines Gesichts eines Benutzers durch eine Geste zu dem Zeitpunkt einer Streaming-Aufnahme in einem nachfolgenden Verarbeiten eines Tutorials eines Gesichtsregistrierungsverarbeitens ausdrückt, das das Gesicht des Benutzers im Voraus registriert;
einen Führungstonsteuerbereich (31), der konfiguriert ist, um eine Ausgabe eines Gestenführungstons gemäß einer Geste zusammen mit dem Gestentreiben zu steuern,
wobei der Führungstonsteuerbereich (31) konfiguriert ist, um eine Ausgabe eines Gesichtsrichtungsführungstons zu steuern, der die Bewegung des Gesichts des Benutzers in der Streaming-Aufnahme führt, die nach dem Tutorial durchgeführt wird;
einen Merkmalsvektorextraktionsbereich (33), der konfiguriert ist, um eine Vielzahl von Merkmalsvektoren aus einem Gesichtsbild des Benutzers aus verschiedenen Winkeln zu extrahieren, das durch ein Ändern der Richtung erfasst wird, in die der Benutzer geführt wird, um das Gesicht gemäß der Führung des Gesichtsrichtungsführungstons zu bewegen, wenn die Streaming-Aufnahme durchgeführt wird; und
einen Zentrumsvektorberechnungsbereich (34), der konfiguriert ist, um einen Zentrumsvektor zu berechnen, der ein Zentrum der Vielzahl von Merkmalsvektoren ist, und den Zentrumsvektor in einer Gesichtsdatenbank zu registrieren.

2. Sich bewegendes Objekt (11) nach Anspruch 1, wobei der Gestensteuerbereich (32) konfiguriert ist, um eine Geschwindigkeit und eine Richtung, mit der der Benutzer geführt wird, das Gesicht zu dem Zeitpunkt der Streaming-Aufnahme zu bewegen, durch eine Geste eines Gesichtsabschnitts (13) des sich bewegenden Objekts (11) auszudrücken.

3. Sich bewegendes Objekt (11) nach Anspruch 2, wobei der Führungstonsteuerbereich (31) konfiguriert ist, um einen konstanten Rhythmus, der der Geschwindigkeit entspricht, als den Gestenführungston auszugeben.

4. Sich bewegendes Objekt (11) nach Anspruch 1, ferner umfassend:
einen Grenzwerteinstellbereich (26), der konfiguriert ist, um einen Grenzwert einzustellen, der in einem Gesichtsauthentifizierungsverarbeiten verwendet wird, das eine Ähnlichkeit mit dem Zentrumsvektor bewertet, der in der Gesichtsdatenbank registriert ist.

5. Sich bewegendes Objekt (11) nach Anspruch 4, wobei der Grenzwerteinstellbereich (26) konfiguriert ist, um als den Grenzwert einen beliebigen von einem ersten Grenzwert, der ein Wert ist, der zu dem Zeitpunkt eines Gestaltens bestimmt wird, einem zweiten Grenzwert, der einer Distanz zu dem Merkmalsvektor an einer am weitesten entfernten Position zentriert auf dem Zentrumsvektor entspricht, und einem dritten Grenzwert, der ein Maximalwert des ersten Grenzwerts und des zweiten Grenzwerts ist, einzustellen.

6. Steuerverfahren, das in der Lage ist, die intuitive Gesichtsregistrierung durchzuführen, umfassend:
Steuern eines Gestentreibens, das eine Bewegung eines Gesichts eines Benutzers durch eine Geste zu dem Zeitpunkt einer Streaming-Aufnahme in dem nachfolgenden Verarbeiten eines Tutorials des Gesichtsregistrierungsverarbeitens ausdrückt, das das Gesicht des Benutzers im Voraus registriert;
Steuern einer Ausgabe eines Gestenführungstons gemäß einer Geste zusammen mit dem Gestentreiben,
wobei der Führungstonsteuerbereich (31) konfiguriert ist, um eine Ausgabe eines Gesichtsrichtungsführungstons zu steuern, der die Bewegung des Gesichts des Benutzers in der Streaming-Aufnahme führt, die nach dem Tutorial durchgeführt wird;
Extrahieren einer Vielzahl von Merkmalsvektoren aus einem Gesichtsbild des Benutzers aus verschiedenen Winkeln, das durch das Ändern der Richtung erfasst wird, in die der Benutzer geführt wird, um das Gesicht gemäß der Führung des Gesichtsrichtungsführungstons zu bewegen, wenn die Streaming-Aufnahme durchgeführt wird; und
Berechnen eines Zentrumsvektors, der ein Zentrum der Vielzahl von Merkmalsvektoren ist, und Registrieren des Zentrumsvektors in einer Gesichtsdatenbank.

## Revendications

1. Objet en mouvement (11) pouvant réaliser un enregistrement de visage intuitif, comprenant :
une section de commande gestuelle (32) configurée pour commander une instruction gestuelle qui exprime un mouvement d'un visage d'un utilisateur par une gestuelle au moment d'une capture en flux continu dans un traitement ultérieur d'un didacticiel de traitement d'enregistrement de visage qui enregistre le visage de l'utilisateur à l'avance ;
une section de commande de son de guidage (31) configurée pour commander une sortie d'un son de guidage gestuel conformément à une gestuelle accompagnée de l'instruction gestuelle,
dans lequel la section de commande de son de guidage (31) est configurée pour commander une sortie d'un son de guidage d'orientation de visage qui guide le mouvement du visage de l'utilisateur dans la capture en flux continu réalisée après le didacticiel ;
une section d'extraction de vecteurs de caractéristiques (33) configurée pour extraire une pluralité de vecteurs de caractéristiques d'une image de visage de l'utilisateur sous divers angles acquis en changeant l'orientation vers laquelle l'utilisateur est guidé pour déplacer le visage conformément à un guidage du son de guidage d'orientation de visage lorsque la capture en flux continu est réalisée ; et
une section de calcul de vecteur central (34) configurée pour calculer un vecteur central qui est un centre de la pluralité de vecteurs de caractéristiques et enregistrer le vecteur central dans une base de données de visages.

2. Objet en mouvement (11) selon la revendication 1, dans lequel la section de commande gestuelle (32) est configurée pour exprimer une vitesse à laquelle et une orientation vers laquelle l'utilisateur est guidé pour déplacer le visage au moment de la capture en flux continu par une gestuelle d'une partie de visage (13) de l'objet en mouvement (11).

3. Objet en mouvement (11) selon la revendication 2, dans lequel la section de commande de son de guidage (31) est configurée pour délivrer un rythme constant correspondant à la vitesse en tant que son de guidage gestuel.

4. Objet en mouvement (11) selon la revendication 1, comprenant en outre :
une section de définition de valeur seuil (26) configurée pour définir une valeur seuil utilisée dans un traitement d'authentification faciale qui évalue une similarité avec le vecteur central enregistré dans la base de données de visages.

5. Objet en mouvement (11) selon la revendication 4, dans lequel la section de définition de valeur seuil (26) est configurée pour définir, en tant que valeur seuil, l'une quelconque parmi une première valeur seuil qui est une valeur déterminée au moment de la conception, une deuxième valeur seuil correspondant à une distance par rapport au vecteur de caractéristique au niveau d'une position la plus éloignée centrée sur le vecteur central, et une troisième valeur seuil qui est une valeur maximale de la première valeur seuil et de la deuxième valeur seuil.

6. Procédé de commande pouvant réaliser un enregistrement de visage intuitif, comprenant :
la commande d'une instruction gestuelle qui exprime un mouvement d'un visage d'un utilisateur par une gestuelle au moment d'une capture en flux continu dans un traitement ultérieur d'un didacticiel de traitement d'enregistrement de visage qui enregistre le visage de l'utilisateur à l'avance ;
la commande d'une sortie d'un son de guidage gestuel conformément à une gestuelle accompagnée de l'instruction gestuelle,
dans lequel la section de commande de son de guidage (31) est configurée pour commander une sortie d'un son de guidage d'orientation de visage qui guide le mouvement du visage de l'utilisateur dans la capture en flux continu réalisée après le didacticiel ;
l'extraction d'une pluralité de vecteurs de caractéristiques d'une image de visage de l'utilisateur sous divers angles acquis en changeant l'orientation vers laquelle l'utilisateur est guidé pour déplacer le visage conformément à un guidage du son de guidage d'orientation de visage lorsque la capture en flux continu est réalisée ; et
le calcul d'un vecteur central qui est un centre de la pluralité de vecteurs de caractéristiques et l'enregistrement du vecteur central dans une base de données de visages.
